# FASCICULE DE BREVET EUROPEEN

(11) **EP 2 883 049 B1**
(45) Date de publication et mention de la délivrance du brevet: **31.05.2017**
(21) Numéro de dépôt: 13727269.6
(22) Date de dépôt: 06.05.2013
(51) Int. Cl.: G01N 33/74, G01N 33/68, G01N 33/53, C07K 16/26

(54) **COMPOSITION DE CONJUGUES ET UTILISATIONS**
ZUSAMMENSETZUNG VON KONJUGATEN UND VERWENDUNGEN
COMPOSITION OF CONJUGATES AND USES

(30) Priorité: 13.08.2012 FR 1257780
(43) Date de publication de la demande: 17.06.2015
(73) Titulaire: Repropharm, 37380 Nouzilly (FR)
(72) Inventeur: DECOURTYE, Jérémy, F-37000 Tours (FR); KARA, Elodie, F-37100 Tours (FR); DUPUY, Laurence, F-37110 Villedomer (FR); MAUREL, Marie-Christine, F-37000 Tours (FR)
(74) Mandataire: Novagraaf Technologies
(86) Numéro de dépôt international: PCT/FR2013/051013
(87) Numéro de publication internationale: WO 2014/027149

(56) Documents cités:
- EP-A2- 0 088 303
- WO-A1-89/05658
- WO-A1-91/10138
- CN-A- 101 097 216
- CN-A- 101 576 559
- RU-C2- 2 202 798
- US-A- 5 670 690
- CONTRERAS-SOLIS I ET AL: "Ovarian and endocrine responses in tropical sheep treated with reduced doses of cloprostenol", ANIMAL REPRODUCTION SCIENCE, ELSEVIER SCIENCE PUBLISHERS, AMSTERDAM, NL, vol. 114, no. 4, 1 septembre 2009 (2009-09-01), pages 384-392, XP026301851, ISSN: 0378-4320, DOI: 10.1016/J.ANIREPROSCI.2008.10.013 [extrait le 2008-11-01]
- A J Vyse ET AL: "Detection of Rubella Virus-Specific Antibody to Fluorescein Isothiocyanate Immunosorbent Assay Using Monoclonal Amplification-Based Enzyme-Linked Immunoglobulin G in Saliva by an", J. Clin. Microbiol., 1 février 1999 (1999-02-01), pages 391-395, XP055065428, Extrait de l'Internet: URL:http://www.ncbi.nlm.nih.gov/pmc/articl es/PMC84317/pdf/jm000391.pdf [extrait le 2013-06-05]
- JOHN R ET AL: "ANTIBODY INTERFERENCE IN TWO-SITE IMMUNOMETRIC ASSAY FOR THYROTROPIN", ANNALS OF CLINICAL BIOCHEMISTRY, vol. 26, no. 4, 1989, pages 346-352, XP002698274, ISSN: 0004-5632
- ANONYMOUS: 'Normal serum | definition of normal serum by Medical dictionary', [en ligne] 01 Janvier 2012, XP055308216 Extrait de l'Internet: <URL:http://medical-dictionary.thefreedicti onary.com/normal+serum> [extrait le 2016-10-06]

## Description

### Domaine technique

La présente invention se rapporte à une composition de conjugués et son utilisation dans un procédé de détection de composés, par exemple de molécules biologiques, par exemple de peptides, de protéines, d'hormones protéiques, glycoprotéiques ou stéroïdes, d'acides nucléiques, d'anticorps dans un échantillon, par exemple un échantillon biologique.

La présente invention se rapporte également à une composition de conjugués et son utilisation dans un procédé de détection du pic préovulatoire de LH dans un échantillon biologique provenant de mammifères et une trousse pour la mise en oeuvre du procédé de détection.

La présente invention trouve une application notamment dans le domaine médical et vétérinaire.

Dans la description ci-dessous, les références entre crochets ([]) renvoient à la liste des références présentée à la fin du texte.

### Etat de la technique

Le cycle de l'ovulation chez les mammifères fait intervenir un procédé hormonal complexe impliquant en particulier les hormones gonadotropes hypophysaires : l'hormone lutéinisante (LH) et l'hormone folliculo-stimulante (FSH).

Il existe actuellement un test de détection de l'ovulation pour la chienne et la chatte basé sur la détection de la LH. Toutefois, ce test n'est pas fiable à 100% et les résultats obtenus comprennent parfois un nombre significatif de faux positifs.

Il existe également, pour les êtres humains de sexe féminin, des tests d'ovulations basés par exemple sur une réaction d'agglutination ou sur une réaction immunoenzymatique. Beaucoup de ces tests se présentent sous forme de bandelette et se réalisent à partir de l'urine dans laquelle ils détectent la présence ou l'absence de la LH. Toutefois, l'interprétation des résultats obtenus peut-être ambigüe, à savoir s'il y a présence ou absence d'une deuxième bande colorée, et induit un nombre significatif de faux positifs. Dans ce cas, ces procédés conduisent à une mauvaise interprétation et ne permettent pas de déterminer avec précision à quel moment va intervenir l'ovulation. CN101097216 écrit une composition comprenant un anticorps couplé à une enzyme (alkaline phosphatase-sheep-anti-human IgG), sérum de veau foetal, glycérol. La composition comprend également du sérum polyclonal en provenance de lapin

US5670690 décrit une composition comprenant un anticorps couplé à une enzyme (Horseradish peroxidase (HRP)-labeled goat anti-rabbit IgG). Cet anticorps est présent dans un tampon de dilution, comprenant du sérum de veau foetal et du glycérol à 15%. Cette composition est ajouté à un échantillon comprenant du sérum de lapin polyclonal.

Contreras-Solis et al (2009. Animal Rep Sci, 14(4), 384 - 392) décrivent une composition comprenant un anticorps couplé à une enzyme et du sérum de veau foetal et son utilisation dans un immunodosage ELISA. De plus, ces tests étant tous urinaires, sont difficilement applicables pour des mammifères animaux tels que les porcins, ovins, bovins, caprins ou nécessitent alors la présence d'une personne pour récolter l'urine du mammifère ce qui dans la pratique n'est pas réalisable et implique des coûts additionnels.

Deux autres types de tests existent pour la femme, l'un est basé sur la mesure du pH de la peau via la sueur par un microprocesseur maintenu en contact étroit avec la peau grâce à un bracelet à scratch porté au poignet. L'autre test est basé sur l'observation, à l'aide d'un petit microscope, de salive séchée qui forme des motifs différents selon le taux d'estrogènes sécrétés au cours d'un cycle menstruel. Il exige de faire l'examen sur de la salive parfaitement propre prélevée à jeûn, ce qui est impossible avec les animaux. Ces deux dispositifs onéreux et nécessitant un équipement particulier, sont inadaptés pour les animaux. De plus, les paramètres mesurés sont moins précis que le pic pré ovulatoire de LH pour une datation fiable de l'ovulation.

En outre les procédés connus dans l'état de la technique pour les êtres humains ne sont pas directement applicables aux autres mammifères, en raison des spécificités différentes des anticorps utilisés et/ou du coût rédhibitoire qu'ils entraînent.

Il existe donc un réel besoin de trouver un procédé et un test palliant ces défauts, inconvénients et obstacles de l'art antérieur, en particulier un procédé permettant de détecter le pic de LH sur tout mammifère avec une sensibilité accrue. En outre il existe un réel besoin de trouver un procédé permettant une interprétation simple du résultat sans ambiguïté et fiable dans le temps et à faible coût.

En particulier, il existe un réel besoin de trouver une composition stable permettant de conserver les réactifs nécessaires à la mise en oeuvre de ces tests, par exemple au réfrigérateur, et à faible coût.

### Description de l'invention

La présente invention a précisément pour but de surmonter les inconvénients de l'art antérieur en fournissant une composition de conjugués comprenant une composition de conjugués comprenant un anticorps couplé à une enzyme, du sérum de veau foetal, de 5 à 50 % en volume de sérum normal de lapin par rapport au volume total de la composition, et de 1 à 30 % en volume par rapport au volume total de la composition de glycérol.

La présente décrit également une composition de conjugués comprenant un anticorps couplé à une enzyme, au moins deux sérums différents de mammifères, et du glycérol.

Selon l'invention, un anticorps couplé à une enzyme peut être un anticorps polyclonal ou monoclonal. Selon l'invention, l'anticorps couplé à une enzyme peut être un anticorps de lapin, d'ovin, de caprin, d'équin, de bovin, de porcin, de souris, de rat.

En d'autres termes, selon l'invention, l'anticorps couplé à une enzyme peut être un anticorps fait chez le lapin, l'ovin, le caprin, l'équin, le bovin, le porcin, la souris, le rat.

Selon l'invention l'anticorps couplé à une enzyme peut être un anticorps dirigé contre une molécule, par exemple une molécule chimique et/ou une molécule biologique, par exemple une protéine, une séquence d'acides aminés, une séquence d'acide nucléique, une hormone glycoprotéique, par exemple l'hormone lutéinisante, l'hormone folliculostimulante, l'hormone de croissance, une hormone stéroïde, par exemple les progestagènes, par exemple la progestérone, par exemple les estrogènes, par exemple l'oestradiol, par exemple les androgènes, par exemple la testostérone.

Selon l'invention, la concentration d'anticorps couplé à une enzyme peut être de 2 à 50 µg/ml.

Selon l'invention l'anticorps peut être couplé à une enzyme choisie dans le groupe comprenant l'enzyme péroxydase, la béta-galactosidase, la glucose oxydase et la phosphatase alcaline. De préférence l'enzyme est la péroxydase, par exemple une péroxydase à hème ou une péroxydase sans hème. Il peut s'agir par exemple d'une peroxydase de raifort commercialisée par la société Sigma sous la référence catalogue P6782.

Dans la présente par sérum différent de mammifères on entend des sérums provenant respectivement de mammifères différents, pouvant être d'espèces différentes ou d'âges différents ou de sexes différents.

Selon l'invention, la concentration desdits au moins deux sérums différents de mammifère peut être comprise de 2 à 75% en volume, de 5 à 50% en volume par rapport au volume total de la composition de la présente invention.

Dans la présente par sérum de mammifère on entend du sérum de mammifère ou du sérum normal de mammifère.

Dans la présente le sérum de mammifère peut être du sérum humain ou animal. Il peut s'agir par exemple de sérum d'un animal d'élevage, d'un animal de compagnie, ou de tout autre animal. Par exemple, l'animal d'élevage peut être choisi dans le groupe comprenant les bovins, porcins, ovins, caprins, camelins, canins, équins, murins, primates. Par exemple, l'animal de compagnie peut être choisi dans le groupe comprenant les canins et les félins. Il peut s'agir par exemple de sérum choisi parmi le sérum de veau foetal, le sérum de lapin, ou un mélange de ceux-ci.

Dans la présente la concentration en volume de sérum de mammifère peut être comprise de 2 à 75% en volume, de 5 à 50% en volume par rapport au volume total de la composition de la présente invention.

Dans la présente par sérum normal de mammifère on entend du sérum obtenu à partir d'un mammifère sain et n'ayant jamais été immunisé contre un antigène particulier.

Dans la présente le sérum normal de mammifère peut être du sérum humain ou animal. Il peut s'agir par exemple de sérum normal d'un animal d'élevage, d'un animal de compagnie, ou de tout autre animal par exemple, les bovins, porcins, ovins, caprins, camelins, canins, équins, murins, félins, primates. Il peut s'agir par exemple de sérum normal choisi parmi le sérum normal de veau foetal, le sérum normal de lapin ou un mélange de ceux-ci. Dans la présente, le sérum de lapin normal peut être du sérum disponible dans le commerce, par exemple il peut s'agir du sérum de lapin normal commercialisé par la société Dutscher 2012 sous la référence catalogue P30-1101 ou du sérum obtenu à partir de lapins n'ayant jamais été immunisés contre un antigène particulier.

Selon l'invention la concentration en volume de sérum normal de lapin est comprise, de 5 à 50% en volume par rapport au volume total de la composition de la présente invention.

Dans la présente, le glycérol peut être du glycérol disponible dans le commerce, par exemple il peut s'agir du glycérol commercialisé par la Société Sigma sous la référence catalogue G7893.

Selon l'invention, la composition de conjugués comprend de 1 à 30% en volume de glycérol par rapport au volume total de la composition, de 1 à 20% en volume de glycérol par rapport au volume total de la composition de la présente invention.

Selon l'invention, la composition de conjugués peut être sous toute forme connue de l'homme du métier. Il peut s'agir, par exemple d'une solution aqueuse, d'une émulsion huile-dans-l'eau, eau-dans-l'huile, une émulsion multiple, une microémulsion, d'une émulsion solide, un gel aqueux ou hydro-alcoolique.

Avantageusement, les inventeurs ont montré de manière surprenante que la composition de conjugués selon l'invention peut être conservée à - 20°C et/ou à 4°C sans dénaturation et/ou perte des propriétés biologiques/physicochimiques de ladite composition et de ses composants. En particulier, les inventeurs ont montré de manière surprenante que la composition selon l'invention permet de conserver l'activité biologique de l'anticorps couplé à une enzyme même si la composition est mise à une température comprise entre -20°C et 4°C.

La présente invention a également pour objet l'utilisation de la composition de conjugués selon l'invention dans un procédé de détection de composés, par exemple de molécules/composés biologiques, par exemple d'acides nucléiques, d'anticorps, de peptides, de protéines, par exemple le facteur de croissance endothéliale vasculaire (VEGF), le facteur de croissance endothéliale (EGF), d'hormones, par exemple d'hormones de croissance, par exemple la somatotropine, d'hormones gonadotropes, par exemple l'hormone lutéinisante (LH), l'hormone folliculo-stimulante (FSH), d'hormones stéroïdes, par exemple les progestagènes, par exemple la progestérone, par exemple les estrogènes, par exemple l'oestradiol, par exemple les androgènes, par exemple la testostérone, dans un échantillon, par exemple un échantillon biologique.

Dans la présente par procédé de détection de composés on entend par exemple un procédé immuno-enzymatique, par exemple un procédé ELISA, et/ou tout procédé de détection de composés, par exemple biologique, connue de l'homme du métier.

Selon l'invention, par « échantillon biologique » on entend un échantillon liquide ou solide. Selon l'invention, l'échantillon peut être tout liquide biologique, par exemple, il peut s'agir d'un échantillon de sang, de plasma, de sérum, de mucus vaginal, de mucus nasal, de salive, d'urine et/ou de lait, de larmes, de sueur. Selon l'invention, l'échantillon peut être un échantillon préalablement prélevé sur ledit mammifère.

Selon l'invention, la composition de conjugués de l'invention peut être également utilisée dans un procédé de détection du pic préovulatoire de LH.

Il peut s'agir par exemple d'un procédé de détection du pic préovulatoire de LH dans un échantillon biologique provenant de mammifères et comprenant les étapes suivantes : a) fixer un anticorps anti-LH sur une surface test ; b) mettre en contact la surface test sur laquelle est fixé ledit anticorps anti-LH avec une solution tampon comprenant de 5 à 50% en volume de sérum de veau foetal ; c) mettre en contact ladite surface obtenue à l'étape (b) avec un échantillon biologique ; d) après l'étape (c) rincer la surface test dans une solution de lavage ; e) mettre en contact la surface test rincée à l'étape (d) avec une solution tampon de conjugués comprenant un anticorps anti-LH couplé à une enzyme et de 5 à 50% en volume de sérum de veau foetal ; f) après l'étape (e) rincer la surface test dans une solution de lavage ; et g) après l'étape (f) mettre en contact la surface test avec une solution comprenant un substrat de ladite enzyme,

Selon l'invention, la solution tampon de conjugué comprend de 5 à 50% en volume, de 5 à 10% en volume, de préférence 5 % en volume de sérum de lapin normal.

Avantageusement, l'utilisation de sérum de lapin normal dans la solution de conjugués permet d'augmenter la sensibilité du procédé de détection du pic de LH. Selon l'invention, la solution tampon de conjugué comprend de 1 à 30% en volume de glycérol, de préférence de 1 à 15% en volume de glycérol.

Avantageusement, l'utilisation de glycérol dans la solution de conjugué permet d'augmenter la sensibilité du procédé de détection du pic de LH .

Avantageusement, lorsque la solution tampon de conjugués comprend en outre du sérum de lapin normal et du glycérol, ladite solution peut être conservée à une température entre 4°C et -20°C sans détérioration des propriétés de l'anticorps couplé à une enzyme.

Selon l'invention, la solution tampon de conjugué peut être préparée préalablement ou concomitamment à la mise en oeuvre du procédé de détection de composés, par exemple du procédé précité.

La présente invention a également pour objet l'utilisation de la composition de conjugués dans une trousse pour la mise en oeuvre d'un procédé de détection de composés, par exemple un procédé tel que décrit ci-dessus.

Avantageusement, la composition de conjugués selon l'invention permet une augmentation de la sensibilité du procédé de détection de composés et de la trousse dans laquelle elle est utilisée.

D'autres avantages pourront encore apparaître à l'homme du métier à la lecture des exemples ci-dessous, illustrés par les figures annexées, données à titre illustratif.

### Brève description des figures

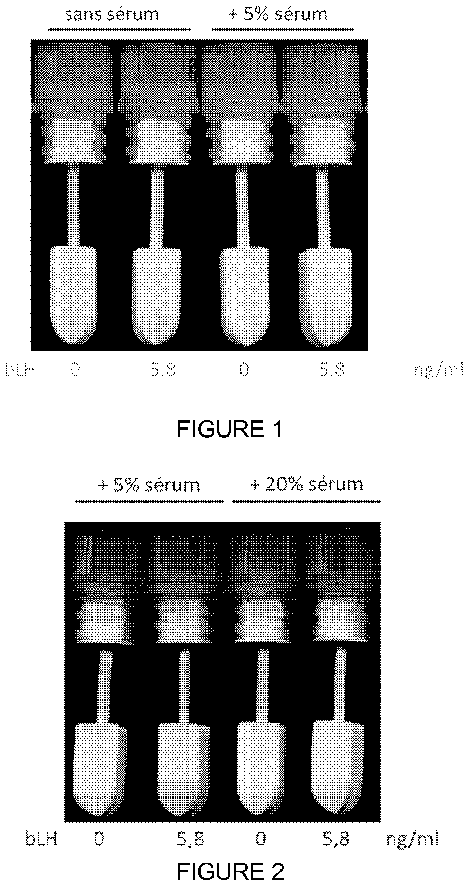

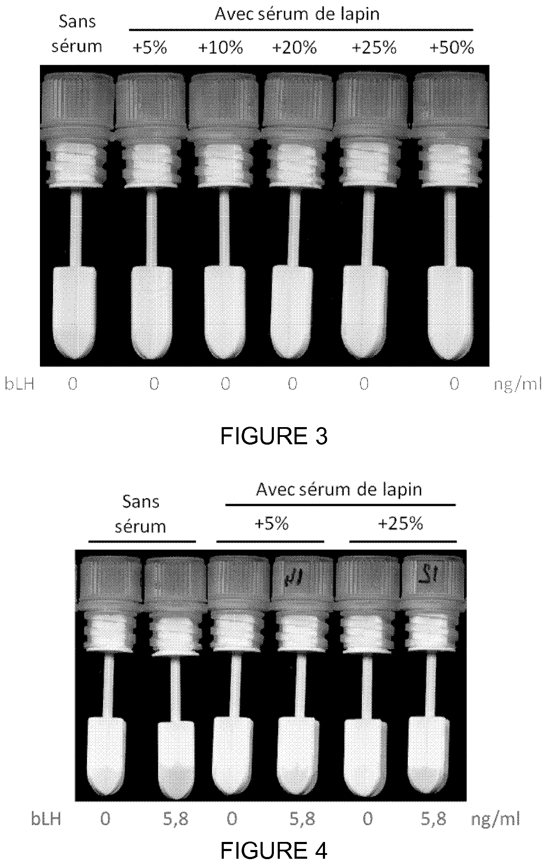

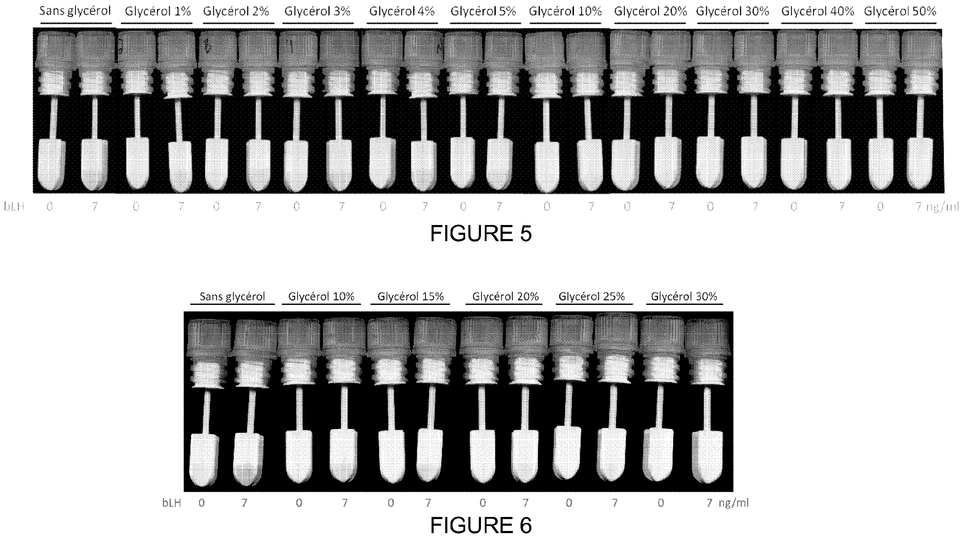

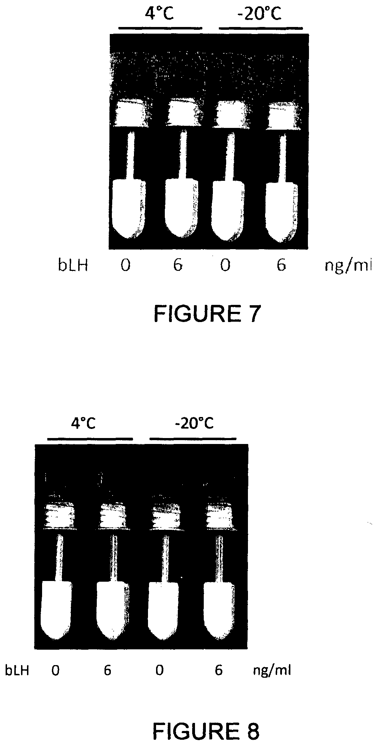

La figure 1 représente une photographie de bâtonnets (« sticks ») après la mise en oeuvre du procédé de l'invention dans lequel le tampon de conjugué comprend ou non 5% de sérum de lapin normal et l'AC2 HRP est à la concentration de 5µg/ml. Des échantillons de plasmas bovins comprenant une concentration de 0 ou 5,8 ng/ml de LH ont été utilisés. La figure 2 représente une photographie de bâtonnets (« sticks ») après la mise en oeuvre du procédé de l'invention dans lequel le tampon de conjugué comprend 5% en volume ou 20% en volume de sérum de lapin normal et l'AC2 HRP est à la concentration de 5µg/ml. Le procédé a été réalisé avec des échantillons de plasmas bovins comprenant une concentration de 0 ou 5,8 ng/ml de LH. La figure 3 représente une photographie de bâtonnets (« sticks ») après la mise en oeuvre du procédé de l'invention dans lequel le tampon de conjugué comprend 5, 10, 20, 25 ou 50 % en volume de sérum de lapin normal et l'AC2 HRP est à la concentration de 5µg/ml. Il a été réalisé avec des échantillons de plasmas bovins comprenant une concentration de 0 ng/ml de LH. La figure 4 représente une photographie de bâtonnets (« sticks ») après la mise en oeuvre du procédé de l'invention dans lequel le tampon de conjugué comprend 0,5 ou 25% en volume de sérum de lapin normal et l'AC2 HRP est à la concentration de 10µg/ml. Le procédé a été réalisé avec des échantillons de plasmas bovins comprenant une concentration de 0 ou 5,8 ng/ml de LH. La figure 5 représente une photographie de bâtonnets (« sticks ») après la mise en oeuvre du procédé de l'invention dans lequel le tampon de conjugué comprend 5% en volume de sérum de lapin normal et 0, 1, 2, 3, 4, 5, 10, 20, 30, 40 ou 50% en volume de glycérol et l'AC2 HRP est à la concentration de 5 µg/ml. Il a été réalisé avec des échantillons de plasmas bovins comprenant une concentration de 0 ou 7 ng/ml de LH. La figure 6 représente une photographie de bâtonnets (« sticks ») après la mise en oeuvre du procédé de l'invention dans lequel le tampon de conjugué comprend 5% en volume de sérum de lapin normal et 10, 15, 20, 25 ou 30% en volume de glycérol et l'AC2 HRP est à la concentration de 10 µg/ml. Il a été réalisé avec des échantillons de plasmas bovins comprenant une concentration de 0 ou 7 ng/ml de LH. La figure 7 représente une photographie de bâtonnets (« sticks ») après la mise en oeuvre du procédé de l'invention dans lequel le tampon de conjugué utilisé a été conservé à -20°C ou 4°C pendant 3 semaines et comprend notamment 5% en volume de sérum de lapin normal et 1% en volume de glycérol et l'AC2 HRP est à la concentration de 5 µg/ml. Il a été réalisé avec des échantillons de plasmas bovins comprenant une concentration de 0 ou 6 ng/ml de LH. La figure 8 représente une photographie de bâtonnets (« sticks ») dans lequel le tampon de conjugué comprend 5% en volume de sérum de lapin normal et 10% en volume de glycérol et l'AC2 HRP est à la concentration de 10µg/ml. Il a été réalisé avec des échantillons de plasmas bovins comprenant une concentration de 0 ou 6 ng/ml de LH.

### EXEMPLES

### Exemple 1 : procédé de fabrication et composition de conjugués comprenant du sérum de veau foetal, du sérum de lapin normal et du glycérol.

Une composition comprenant des anticorps couplés à une enzyme (conjugués) a été préparée comme suit :
Le procédé de préparation de l'anticorps anti-LH couplé à la péroxydase de raifort (HRP ; fournisseur Sigma, référence P6782), dénommé ci-dessous AC2 HRP à 10µg/ml comprend les étapes suivantes ;
   **1-** une quantité d'AC2 HRP nécessaire pour avoir une concentration finale de 10 µg/ml a été ajoutée et incubée dans un volume de sérum de lapin normal correspondant à 5% du volume de la préparation finale.
   **2-** le mélange a été ensuite incubé 45 minutes à 37°C.
   **3-** un volume nécessaire a été ajouté de PBS SVF 50% (fournisseur Lonza, référence 14-801 F) contenant 0,05% en volume de ProClin300 (marque déposée, fournisseur Sigma-Aldrich, référence 48912-U), et 10% en volume de glycérol (fournisseur Sigma, référence G7893), afin d'obtenir une solution d'AC2 HRP à une concentration finale de 10µg/ml.
   **4-** le mélange obtenu à l'étape 3 a été ensuite incubé 15 minutes à 37°C et 300 µl du mélange incubé a été distribué par cryotube (cryotube NUNC, Dutscher, ref. 055003).
   **5-** conservation à 4°C avec les autres composants du test

En d'autres termes, pour un volume final de 3ml d'une solution d'AC2 HRP, c'est-à-dire un exemple de composition de conjugués, à 10µg/ml :
**1-** 30µg d'AC2 HRP ont été incubés dans 150µl de sérum de lapin
**2-** incubation 45 minutes à 37°C
**3-** ajout de 2,85 ml de PBS SVF 50% en volume contenant 0,05% en volume de ProClin300, et 10% en volume de glycérol.
**4-** incubation 15 minutes à 37°C et distribution de 300 µl par cryotube.
**5-** conservation à 4°C.

Le sérum de lapin normal utilisé à l'étape 1 a été obtenu par prélèvement de sang sur des lapins non immunisés : on parle de sérum de lapin normal. Il peut être aussi acheté dans le commerce (fournisseur Dutscher 2012 sous la référence catalogue P30-1101). La composition du PBS était K₂HPO₄ 0,01 M (fournisseur VWR ; référence 26930.293), KH₂PO₄ 0,01 M (fournisseur VWR ; référence 26936.293), NaCl 0,15M (fournisseur VWR; référence 27810.295), pH 7.4. Le PBS est préparé dans de l'eau milliQ et filtré sur filtre de porosité 0,22µm (fournisseur Millipore ; référence GSWP04700).

Un anticorps anti-LH couplé à la péroxydase (AC2 HRP) a été préparé à 5µg/ml selon le procédé décrit ci-dessus dans 5% en volume de sérum de lapin normal, puis dilué dans du PBS SVF 50% (fournisseur Lonza, référence 14-801 F) en volume contenant 0,05% en volume de ProClin300 (marque déposée, fournisseur Sigma-Aldrich, référence 48912-U) et comprenant en outre de 1% à 50% en volume de glycérol (fournisseur Sigma, référence G7893).

### Exemple 2 : mise en oeuvre d'un procédé de détection de molécules avec une solution de conjugués (AC2 HRP) comprenant ou non pas 5% de sérum de lapin normal

Dans les exemples 2 à 9, le bâtonnet («stick») est celui commercialisé par la société NUNC (Immuno™Stick Nunc, Maxisorp), le ProClin300 (marque déposée) est commercialisé par la société Sigma-Aldrich, ref. 48912-U et le Sérum de Veau Foetal (SVF) provennait de la société Lonza, sous la référence 14-801 F, le TMB membrane est le TMB membrane commercialisé par le fournisseur KPL sous la référence 50-77-18.

Dans cet exemple, le procédé de détection est un procédé de détection du pic de LH. La surface test utilisée était une surface plastique d'un bâtonnet («stick»). Sur la surface test, un anticorps anti-LH (AC1) a été fixé par recouvrement (coating) avec 250 µl d'anticorps lapin anti-LH bovine obtenu après purification sur colonne de Protéine A Sepharose à partir d'un sérum de lapin immunisé avec de la LH bovine purifiée dénommé ci-dessous AC1 préparé à 20 µg/ml dans du tampon NaHCO₃/Na₂CO₃ 0,1 M pH 9,6 contenant 0,05% en volume de ProClin300. La surface a ensuite été incubée pendant 1 heure à 37°C, puis 18 heures à 4°C. La surface a ensuite été essorée par secousses pour éliminer le reste du liquide d'AC1. La surface test sur laquelle a été fixé l'anticorps a été mise en contact, via le recouvrement (« surcoating ») de la surface avec 900µl de PBS additionné de Sérum de Veau Foetal (SVF) 50% en volume pendant 1 heure à 37°C. Ladite surface obtenue a été ensuite essorée par secousses et séchée à la verticale dans une étuve à 37°C pendant 3 heures.

La surface obtenue a été mise en contact avec un échantillon de 250µl de sang par immersion dans un tube hépariné (cryotube en polypropylène NUNC, ref. 368632). Le tube hépariné a été préalablement traité par 12 µl d'une solution d'héparine à 500Ul/ml préparée à partir de l'Héparine Choay commercialisée par Sanofi Aventis et diluée dans du PBS filtré sur un filtre de 0,2µm de porosité (Millipore, ref. GSWP04700), puis séché pendant 3 heures dans une étuve à 37°C. La surface test a été ensuite rincée dans 30 mL d'une solution de lavage, à savoir une solution tampon phosphate salin PBS pH 7,4 K₂HPO₄/KH₂PO₄ 0,01M - NaCl 0,15M comprise dans un tube de 40 ml. La surface test rincée obtenue a été mise en contact dans un cryotube (commercialisé par la société NUNC, ref. 368632) avec 300µl d'une solution tampon de conjugué comprenant un anticorps de cheval anti-LH ovine couplé à la péroxydase de raifort (HRP) préparé selon le procédé décrit dans « Techniques Immuno-enzymatiques » (Thérèse Ternynck et Stratis Avrameas, éditions INSERM, 1987) dénommé ci-dessous AC2 HRP à 10µg/ml dans du PBS SVF 50% en volume contenant 0,05% en volume de ProClin300 (marque déposée) préalablement préparé.

Dans les conditions dites « sans sérum », l'AC2 HRP a été préparé à 5µg/ml dans une solution PBS SVF 50% en volume contenant 0,05% en volume de ProClin300.

Dans les conditions dites « avec 5% de sérum de lapin », l'AC2 HRP 5µg/ml a d'abord été incubé pendant 45 minutes à 37°C dans un volume de sérum de lapin normal correspondant à 5% du volume final de l'AC2 HRP, puis dilué dans du PBS SVF 50% contenant 0,05% en volume de ProClin300.

Dans chacun des cas, deux plasmas de vache à 0 ou 5,8 ng/ml de LH ont été utilisés.

La surface test a été ensuite rincée dans 30 mL d'une solution de lavage, à savoir une solution tampon phosphate salin PBS pH 7,4 K₂HPO₄/KH₂PO₄ 0,01M - NaCl 0,15M comprise dans un tube de 40 ml. La surface test rincée obtenue a été mise en contact pendant 15 minutes dans un cryotube commercialisé par la société NUNC, ref. 368632 avec une solution comprenant un substrat de péroxydase de raifort (HRP) à savoir 300µL de TMB Membrane. La surface obtenue a été ensuite retirée du tube et l'observation visuelle de la coloration bleue de la surface test a été effectuée et a montré la présence de LH dans l'échantillon testé. Dans cet exemple la quantification du signal coloré a été réalisée comme décrit dans les exemples ci-dessus, c'est-à-dire que les bâtonnets (« sticks ») ont été scannés à l'aide d'un Scanner EPSON (Perfection 1200 PHOTO) puis l'intensité de la couleur obtenue sur chacun d'eux est quantifiée par densitométrie avec le logiciel « Scion Image » (Scion Incorporation). Cette quantification est exprimée en unités de densité. La figure 1 est une photographie des sticks après révélation.

**Tableau 1 : Intensité de la coloration obtenue en fonction de la présence ou absence de sérum de lapin normal dans la solution de conjugué**

| | **Sans sérum** | | **+ 5% sérum de lapin** | |
|---|---|---|---|---|
| LH (ng/ml) | 0 | 5,8 ng/ml | 0 | 5,8 ng/ml |
| Quantification du signal coloré | 11,43 | 26,4 | 4,97 | 24,85 |

Le procédé de préparation de l'AC2 HRP dans 5% de sérum de lapin normal permet de diminuer le bruit de fond, sans modifier l'intensité du signal obtenu avec le plasma à 5,8 ng/ml.

En d'autres termes, la mise en oeuvre d'un procédé de détection du pic de LH utilisant une solution tampon de conjugué comprenant un anticorps anti-LH couplé à une enzyme, à 5µg/ml, 50%de sérum de veau foetal et 5% de sérum de lapin normal permet avantageusement d'augmenter la sensibilité de détection en diminuant le signal non-spécifique.

### Exemple 3 : Essais comparatifs de l'ajout de 5 à 20% de sérum de lapin normal dans l'AC2 HRP préparé à 5µg/ml

L'AC2 HRP a été préparé à 5 µg/ml dans 5% ou 20% de sérum de lapin normal puis dilué dans du PBS SVF 50% en volume contenant 0,05% en volume de ProClin300, afin de comparer l'effet du sérum de lapin normal sur le bruit de fond et le signal obtenu après incubation du stick dans deux plasmas de vache à 0 et 5,8 ng/ml de LH respectivement. Dans cet exemple la quantification du signal coloré a été réalisée comme décrit dans l'exemple 2 ci-dessus. La figure 2 est une photographie des sticks après révélation.

**Tableau 2: Intensité de la coloration obtenue en fonction de la quantité de sérum de lapin normal dans la solution de conjugué**

| | **+ 5% de sérum** | | **+20% sérum** | |
|---|---|---|---|---|
| LH (ng/ml) | 0 | 5,8 ng/ml | 0 | 5,8 ng/ml |
| Quantification du signal coloré | 10,28 | 26,38 | 10,28 | 24,26 |

L'augmentation du pourcentage de sérum de lapin utilisé pour préparer l'AC2 HRP ne modifie pas l'intensité du signal obtenu sur le stick. La préparation de l'AC2 HRP dans 5% ou 20% de sérum de lapin normal donne un résultat équivalent. En d'autres termes, la mise en oeuvre d'un procédé de détection du pic de LH utilisant l'une ou l'autre des solutions tampon de conjugué ne modifie pas la détection du pic de LH.

### Exemple 4: Essais comparatifs de l'ajout de 5 à 50% de sérum de lapin normal dans l'AC2 HRP sur le signal non spécifique d'un procédé de détection de composés

L'AC2 HRP a été préparé à 5µg/ml selon le procédé décrit dans l'exemple 1 ci-dessus dans 5%, 10%, 20%, 25% et 50% en volume de sérum de lapin normal, puis dilué dans du PBS SVF 50% contenant 0,05% de ProClin300. Les sticks ont été incubés dans 250 µl de plasma de vache contenant 0 ng/ml de LH afin de comparer l'effet des différents pourcentages de sérum de lapin normal sur le signal non-spécifique c'est-à-dire le bruit de fond coloré observé sur le stick en absence de toute LH dans le plasma. Dans cet exemple la quantification du signal coloré a été réalisée comme décrit dans l'exemple 2 ci-dessus. La figure 3 est une photographie des sticks après révélation.

**Tableau 3 : Intensité de la coloration obtenue en fonction de la présence ou absence de sérum de lapin normal dans la solution de conjugué**

| | **Sans sérum** | **+5%** | **+10%** | **+20%** | **+25%** | **+50%** |
|---|---|---|---|---|---|---|
| LH (ng/ml) | 0 | 0 | 0 | 0 | 0 | 0 |
| Quantification du signal coloré | 16,66 | 9,89 | 10,08 | 8,61 | 10,12 | 9,08 |

L'ajout de 5% à 50% de sérum de lapin normal dans l'AC2 HRP diminue le bruit de fond sur le stick et permet d'améliorer la sensibilité du test par rapport à une solution de conjugué ne contenant pas de sérum de lapin normal.. En d'autres termes, la mise en oeuvre d'un procédé de détection du pic de LH utilisant une composition de conjugués selon l'invention diminue le signal non spécifique. La concentration de 5% a été retenue pour la réalisation des exemples 5 à 8.

### Exemple 5: Essais comparatifs de l'ajout de 5 à 25% de sérum de lapin normal dans l'AC2 HRP préparé à 10µg/m)

L'AC2 HRP a été préparé à 10µg/ml selon le procédé décrit dans l'exemple 1 ci-dessus dans 5% ou 25% en volume de sérum de lapin normal, puis dissous dans du PBS SVF 50% contenant 0,05% de ProClin300. Les sticks ont été incubés dans 250 µl de plasma de vache contenant 0 ng/ml ou 5,8 ng/ml de LH puis dans l'AC2 HRP et enfin dans le TMB membrane, afin de comparer l'effet des différents pourcentages de sérum de lapin normal sur le bruit de fond et le signal obtenus sur les sticks. Dans cet exemple la quantification du signal coloré a été réalisée comme décrit dans l'exemple 2 ci-dessus. La figure 4 est une photographie des sticks après révélations

**Tableau 4 : Intensité de la coloration obtenue en fonction de la présence ou absence de sérum de lapin normal dans la solution de conjugué**

| | **Sans sérum** | | **+5%** | | **+25%** | |
|---|---|---|---|---|---|---|
| LH (ng/ml) | 0 | 5,8 ng/ml | 0 | 5,8 ng/ml | 0 | 5,8 ng/ml |
| Quantification du signal coloré | 24,25 | 44,82 | 7,3 | 33,35 | 10,28 | 39,13 |

L'augmentation du pourcentage de sérum de lapin normal utilisé pour préparer l'AC2 HRP à 10µg/ml ne modifie pas l'intensité du signal obtenu sur le stick avec un plasma concentré en LH mais réduit le signal non-spécifique obtenu avec un plasma à 0ng/ml. La préparation de l'AC2 HRP dans 5% ou 25% de sérum de lapin normal donne un résultat équivalent.

En d'autres termes, la mise en oeuvre d'un procédé de détection du pic de LH utilisant une solution tampon de conjugué comprenant un anticorps anti-LH couplé à une enzyme, 50% de sérum de veau foetal et 5% ou 25% de sérum de lapin normal augmente la sensibilité de détection.

### Exemple 6 : Essais comparatifs de l'ajout de sérum de lapin normal et de glycérol dans l'AC2 HRP préparé à 5µg/ml

L'AC2 HRP a été préparé à 5µg/ml selon le procédé décrit dans l'exemple 1 ci-dessus dans 5% en volume de sérum de lapin normal, puis dilué dans du PBS SVF 50% en volume contenant 0,05% en volume de ProClin300 et comprenant en outre de 1% à 50% en volume de glycérol en volume (fournisseur Sigma, référence G7893). Les sticks ont été incubés dans 250 µl de plasma de vache contenant 0 ng/ml ou 7 ng/ml de LH puis dans l'AC2 HRP et enfin dans le TMB membrane, afin de comparer l'effet des différents pourcentages de glycérol sur le bruit de fond et le signal présents sur les sticks). La quantification du signal coloré a été réalisée comme décrit dans l'exemple 2 ci-dessus. La figure 5 est une photographie des sticks après révélation.

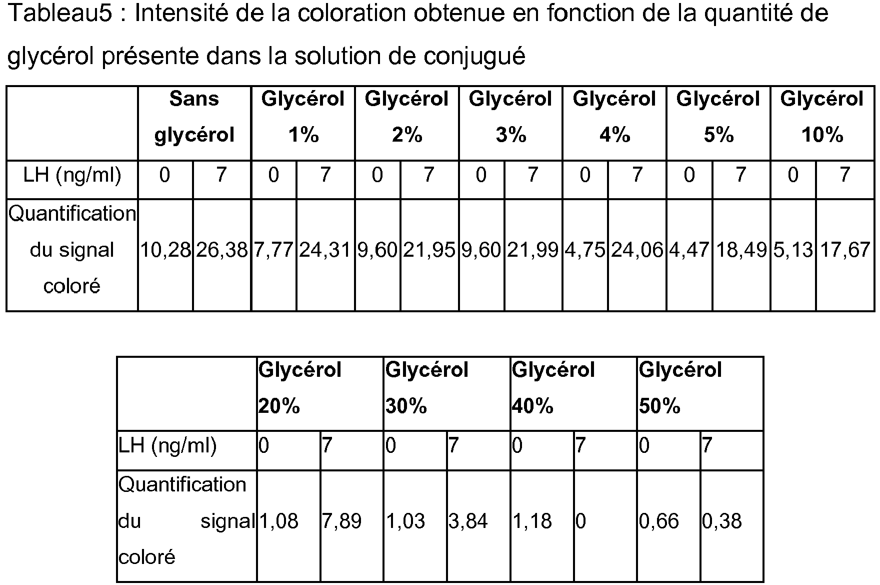

A 5µg/ml d'AC2 HRP, l'addition de 1% à 4% de glycérol diminue le bruit de fond, sans diminuer le signal. Au-delà de 5%, le glycérol atténue non seulement le bruit de fond, mais également le signal spécifique. En d'autres termes, la mise en oeuvre d'un procédé de détection du pic de LH utilisant une solution tampon de conjugué comprenant un anticorps anti-LH couplé à une enzyme, 50% en volume de sérum de veau foetal,5% de sérum de lapin normal et de 1 à 4% de glycérol diminue le bruit de fond sans modifier la détection du pic de LH.

### Exemple 7 Essais comparatifs de l'ajout de sérum de lapin normal et de glycérol dans l'AC2 HRP préparé à 10µg/m)

L'AC2 HRP a été préparé à 10µg/ml selon le procédé décrit dans l'exemple 1 ci-dessus dans 5% en volume de sérum de lapin normal, et complété avec une solution PBS SVF 50% en volume contenant 0,05% en volume de ProClin300 et 10% à 50% en volume de glycérol (fournisseur Sigma, référence G7893). Les sticks ont été incubés dans 250 µl de plasma de vache contenant 0 ng/ml ou 7 ng/ml de LH puis dans l'AC2 HRP et enfin dans le TMB membrane, afin de comparer l'effet des différents pourcentages de glycérol sur le bruit de fond et le signal présents sur les sticks. Dans cet exemple la quantification du signal coloré a été réalisée comme décrit dans l'exemple 2 ci-dessus. La figure 6 est une photographie des sticks après révélation.

**Tableau 6 : Intensité de la coloration obtenue en fonction de la quantité de glycérol présente dans la solution de conjugué**

| | **Sans glycérol** | | **Glycérol 10%** | | **Glycérol 15%** | | **Glycérol 20%** | | **Glycérol 25%** | | **Glycérol 30%** | |
|---|---|---|---|---|---|---|---|---|---|---|---|---|
| LH (ng/ml) | 0 | 7 | 0 | 7 | 0 | 7 | 0 | 7 | 0 | 7 | 0 | 7 |
| Quantification du signal coloré | 14,5 | 51,87 | 6,68 | 30,6 | 6,52 | 28,81 | 4,95 | 17,95 | 4,06 | 15,67 | 2,28 | 10,85 |

A 10µg/ml d'AC2 HRP, l'ajout de 10% ou 15% de glycérol diminue le bruit de fond, tout en atténuant le signal. Au-delà de 15% en volume de glycérol l'extinction du signal spécifique est trop importante rendant impossible la détection visuelle d'un pic de LH. La meilleure sensibilité du test est obtenue avec l'AC2 HRP préparé à 10µg/ml dans 5% de sérum de lapin normal et 10% ou 15% en volume de glycérol. En d'autres termes, la mise en oeuvre d'un procédé de détection du pic de LH utilisant une solution tampon de conjugué comprenant un anticorps anti-LH couplé à une enzyme et 50% en volume de sérum de veau foetal et 5% en volume de sérum de lapin normal et 10% ou 15% de glycérol augmente la sensibilité de détection.

### Exemple 8 : Essais comparatifs dans le cas d'une conservation à 4°C ou -20°C de l'AC2 HRP préparé à 5µg/ml

L'AC2 HRP a été préparé à 5µg/ml selon le procédé décrit dans l'exemple 1 ci-dessus dans 5% en volume de sérum de lapin normal, et complété avec une solution PBS SVF 50% en volume contenant 0,05% en volume de ProClin300 et 1% en volume de glycérol (fournisseur Sigma, référence G7893), et conservé à 4°C ou à -20°C pendant 3 semaines. Au bout de 3 semaines, les sticks ont été incubés dans 250 µl de plasma de vache à 0 ng/ml ou 7 ng/ml de LH, puis dans l'AC2 HRP conservé à 4°C ou à -20°C, et enfin dans le TMB membrane, afin de comparer la conservation de l'AC2 HRP à 4°C et à -20°C. Dans cet exemple la quantification du signal coloré a été réalisée comme décrit dans l'exemple 2 ci-dessus. La figure 7 est une photographie des sticks après révélation.

**Tableau 7: Intensité de la coloration obtenue en fonction de la température de conservation de la solution de conjugué**

| | **4°C** | | **-20°C** | |
|---|---|---|---|---|
| LH (ng/ml) | 0 | 6 | 0 | 6 |
| Quantification du signal coloré | 5,35 | 27,75 | 3,27 | 28,72 |

L'AC2 HRP préparé à 5µg/ml dans 5% en volume de sérum de lapin normal et 1% en volume de glycérol et conservé à 4°C ou à -20°C présente les mêmes performances : on obtient la même intensité de couleur sur les sticks. Cet exemple démontre donc clairement que la composition selon l'invention permet avantageusement de conserver une composition de conjugués entre -20 et 4°C. En outre, la composition selon l'invention permet avantageusement de conserver l'activité biologique des anticorps/enzymes inclus dans la composition.

### Exemple 9 : Essais comparatifs dans le cas d'une conservation à 4°C ou -20°C de l'AC2 HRP préparé à 10µg/ml

L'AC2 HRP a été préparé à 10µg/ml selon le procédé décrit dans l'exemple 1 ci-dessus dans 5% en volume de sérum de lapin normal, et complété avec une solution PBS SVF 50% en volume contenant 0,05% en volume de ProClin300 et 10% en volume de glycérol (fournisseur Sigma, référence G7893), et conservé à 4°C ou à -20°C pendant 3 semaines. Au bout de 3 semaines, les sticks ont été incubés dans 250 µl de plasma de vache contenant 0 ng/ml ou 7 ng/ml de LH, puis dans l'AC2 HRP conservé à 4°C ou à -20°C, et enfin dans le TMB membrane, afin de comparer la conservation de l'AC2 HRP à 4°C et à -20°C. Dans cet exemple la quantification du signal coloré a été réalisée comme décrit dans l'exemple 2 ci-dessus. La figure 8 est une photographie des sticks après révélation.

**Tableau 8 : Intensité de la coloration obtenue en fonction de la température de conservation de la solution de conjugué**

| | **4°C** | | **-20°C** | |
|---|---|---|---|---|
| LH (ng/ml) | 0 | 6 | 0 | 6 |
| Quantification du signal coloré | 3,27 | 20,92 | 2,6 | 20,58 |

L'AC2 HRP préparé à 10µg/ml dans 5% en volume de sérum de lapin normal et 1% en volume de glycérol et conservé à 4°C ou à -20°C présente les mêmes performances : on obtient la même intensité de couleur sur les sticks.

Tel que démontré dans cet exemple le milieu de préparation de l'AC2 HRP permet donc une conservation de l'anticorps conjugué à 4°C aussi efficace qu'un stockage à -20°C.

### Exemple 10: Performances d'une trousse de détection du pic préovulatoire de LH conservée à 4°C avec l'AC2 HRP préparé dans le milieu décrit et revendiqué

L'effet du milieu a été testé dans le cas où l'ensemble des éléments de la trousse fabriquée selon le procédé ci-dessous aient été conservés à 4°C pendant 10 semaines. Dans cet exemple, la trousse comprend un bâtonnet (« stick ») comprenant des surfaces tests, prêt à l'emploi dans un tube hépariné, un tube comprenant la solution de conjugué, à savoir une solution tampon avec un anticorps anti-LH couplé à la péroxydase de raifort (anticorps conjugué), et un tube contenant une solution comprenant le substrat de la péroxydase de raifort, à savoir le TMB membrane appelé substrat. Le matériel utilisé était des immunosticks commercialisés par la société NUNC, et trois cryotubes à bouchon à vis commercialisés par la société NUNC.

Un tube hépariné (a été préparé selon le procédé décrit dans l'exemple 2

Les bâtonnets ont été préparés par recouvrement (« coating ») avec 250 µl d'anticorps lapin anti-LH bovine purifié sur colonne de Protéine A Sépharose à partir d'un immun sérum produit chez le lapin par le prestataire Eurogentec (Belgique) selon le procédé «Standard anti-protein packages 28-day Speedy in Rabbit» (marque de commerce) dénommé ci-dessous AC1 préparé à 20 µg/ml dans du tampon NaHCO₃/Na₂CO₃ 0,1 M pH 9,6 contenant 0,05% en volume de ProClin300. Dans un autre mode de réalisation l'anticorps anti-LH (AC1) était un anticorps de lapin anti-LH porcine il s'agit de l'anticorps produit, par exemple par immunisation de lapins selon le procédé décrit par exemple dans la référence bibliographique Pelletier J., et al., 1968 [5]. Il s'agissait du procédé comprenant par exemple trois injections de 200 µg de LH porcine purifiée commercialisée par la société Tucker Endocrine Research Institute LLC TUENRE (Atlanta, USA), qui ont été réalisées toutes les deux semaines, suivi de dix rappels avec 100µg de LH porcine purifiée effectués toutes les cinq semaines. Les saignées ont été réalisées six et neuf jours après chaque rappel et 40 ml de sang ont été récupérés. A partir des échantillons de sang les anticorps de lapin ont été purifiés par chromatographie d'affinité sur gel de Protéine A sépharose selon le procédé décrit dans « Techniques Immuno-enzymatiques » Thérèse Ternynck et al., éditions INSERM, 1987 [1] Le recouvrement du stick par l'anticorps a été réalisé selon le procédé décrit dans Thérèse Ternynck et al. 1987 [1] Incubation une heure à 37°C, puis 18 heures à 4°C. Essorage du bâtonnet par secousses. Recouvrement (« surcoating ») du bâtonnet dans 900 µl de PBS additionné de Sérum de veau foetal (SVF) 50% pendant 1 heure à 37°C. Essorage du bâtonnet par secousses. Séchage du bâtonnet à la verticale à l'étuve 37°C pendant 3 heures. Conservation du bâtonnet à 4°C dans un tube hépariné séché.

L'anticorps cheval anti-LH ovine couplé à la péroxydase de raifort (HRP), dénommé ci-dessous AC2 HRP, a été préparé à 5 ou 10µg/ml dans du PBS SVF 50% en volume contenant 0,05% en volume de ProClin300, incubation 50 minutes à 37°C. Trois conditions ont été étudiées : a) AC2 HRP préparé à 5µg/ml dans 5% de sérum de lapin normal et 1% de glycérol, b) AC2 HRP préparé à 5µg/ml dans 5% de sérum de lapin normal et 3% de glycérol, et c) AC2 HRP préparé à 10µg/ml dans 5% de sérum de lapin normal et 15% de glycérol.

L'anticorps AC2 HRP a été produit tel que décrit dans l'exemple 2. Dans un autre mode de réalisation il s'agit de l'anticorps produit, par exemple par immunisation de lapins selon le procédé décrit dans la référence bibliographique Pelletier J. et al. 1968 [5]. Il s'agissait du procédé comprenant par exemple trois injections de 200 µg de LH porcine purifiée provenant de la société Tucker Endocrine Research Institute LLCTUENRE (Atlanta, USA), qui ont été réalisées toutes les deux semaines, suivi de dix rappels avec 100µg de LH purifiée effectués toutes les cinq semaines. Les saignées ont été réalisées six et neuf jours après chaque rappel et 40ml de sang ont été récupérés. A partir des échantillons de sang les anticorps de lapin ont été purifiés par chromatographie d'affinité sur gel de Protéine A sépharose selon le procédé décrit dans Thérèse Ternynck et al. 1987 [1]. Le couplage de l'anticorps a été réalisé selon le procédé décrit dans Thérèse Ternynck et al. 1987 [1]. La LH porcine était commercialisée par Tucker Endocrine Research Institute LLCTUENRE (Atanta, USA). La péroxydase utilisée est commercialisée par Sigma sous la référence P6782. La préparation des tubes de révélateur (tube n°3) a été faite en distribuant 300µl de TMB membrane par cryotube. Pour évaluer l'efficacité du milieu, les performances des trousses stockées 10 semaines à 4°C ont été comparées avec celles obtenues juste après la fabrication des trousses, avant tout stockage à 4°C. Dans cet exemple la quantification du signal coloré a été réalisée comme décrit dans l'exemple 2 ci-dessus. Les résultats sont présentés dans le tableau 9 ci-dessous :

**Tableau 9 Intensité de la coloration obtenue en fonction de la conservation ou non de la trousse pour la mise en oeuvre du procédé selon l'invention**

| Quantification de la couleur des sticks | AC2 5µg/ml dans 5% sérum de lapin et 1% glycérol | | AC2 5µg/ml dans 5% sérum de lapin et 3% glycérol | | AC2 10µg/ml dans 5% sérum de lapin et 15% glycérol | |
|---|---|---|---|---|---|---|
| | En sortie de production | Stockage 10 semaines à 4°C | En sortie de production | Stockage 10 semaines à 4°C | En sortie de production | Stockage 10 semaines à 4°C |
| LH 0ng/ml | 10,83 | 7,18 | 11,32 | 6,61 | 9 | 9,17 |
| LH 6ng/ml | 30,76 | 30,71 | 24,36 | 21,03 | 19,17 | 19,74 |

Il a été observé que dans les trois conditions de préparation de l'AC2 HRP, l'intensité du signal coloré du stick, obtenue avec un plasma à 0ng/ml de LH ou avec un plasma à 6ng/ml de LH, n'est statistiquement pas différente avec une trousse en sortie de production ou conservée à 4°C pendant 10 semaines. Ces résultats démontrent donc clairement que la composition dudit milieu de préparation de l'AC2 HRP permet une conservation de la trousse à 4°C sans altération des performances du test tant d'un point du vue de sa sensibilité que de sa stabilité.

L'exemple a été également réalisé avec une composition comprenant l'AC2 HRP à 20µ/ml, préparé dans du PBS SVF 50% contenant 5% de sérum de lapin normal et 10% de glycérol. La quantification du signal coloré obtenu a été réalisée sur des bâtonnets en sortie de production et après 5 mois de conservation à 4°C.

**Tableau 10 Intensité de la coloration obtenue en fonction de la conservation ou non de la trousse pour la mise en oeuvre du procédé selon l'invention**

| Quantification de la couleur des sticks | AC2 20µg/ml dans 5% sérum de lapin et 10% glycérol | |
|---|---|---|
| | En sortie de production | Stockage 5 mois à 4°C |
| LH 0ng/ml | 12,6 | 1,7 |
| LH 6ng/ml | 39,24 | 37,95 |

Il a été observé que l'intensité du signal coloré du stick, obtenue avec un plasma à 0ng/ml de LH ou avec un plasma à 6ng/ml de LH, n'est statistiquement pas différente entre une trousse en sortie de production et une trousse conservée à 4°C pendant 5 mois. Ces résultats démontrent donc clairement que la composition dudit milieu de préparation de l'AC2 HRP permet une conservation de la trousse à 4°C sans altération des performances du test tant d'un point du vue de sa sensibilité que de sa stabilité.

### Listes des références

1. «Techniques Immuno-enzymatiques » Thérèse Ternynck et Stratis Avrameas, éditions INSERM, 1987;
2. Immunobiologie Charles A. Janeway, Paul Travers, Pierre L. Masson - 2003 - Medical ; Janeway's Immunobiology, Kenneth Murphy, Paul Travers, Mark Walport, 2011, éditions GS)
3. « Enzyme-linked immunosorbent assay (ELISA). Quantitative assay of immunoglobulin G », Engvall, E. et Perlman, P., Immunochemistry, 1971 Sep;8(9):871-4 PMID: 5135623
4. « Enzyme-Linked Immunosorbent Assay » Goldsby, R.A., Kindt, T.J., Osborne, B.A. et Kuby, J., in Immunology, 5ème édition (2003), pp. 148-150., W. H. Freeman, New York.
5. « Dosage radio-immunologique de l'hormone lutéinisante plasmatique chez le mouton. Mise au point de la technique de dosage », Pelletier J., Kann G., Dolais J., Rosselin G., C. R. Acad. Sc. Paris, 1968 juin ;266 :2291-2294

## Revendications

1. Composition de conjugués comprenant un anticorps couplé à une enzyme, du sérum de veau foetal, de 5 à 50 % en volume de sérum normal de lapin par rapport au volume total de la composition, et de 1 à 30 % en volume de glycérol par rapport au volume total de la composition.

2. Composition de conjugués selon la revendication 1, ladite composition étant sous une forme choisie parmi une solution aqueuse, une émulsion huile-dans-l'eau, eau-dans-l'huile, une émulsion multiple, une microémulsion, d'une émulsion solide, un gel aqueux ou hydro-alcoolique

3. Utilisation d'une composition selon l'une quelconque des revendications 1 à 2 dans un procédé de détection de composés.

4. Utilisation d'une composition selon la revendication 3 dans une trousse pour la mise en oeuvre d'un procédé de détection de composés biologiques.

## Patentansprüche

1. Konjugatzusammensetzung, umfassend einen Antikörper, der mit einem Enzym gekoppelt ist, fötales Kälberserum, 5 bis 50 Vol.-% normales Kaninchenserum in Bezug auf das Gesamtvolumen der Zusammensetzung sowie 1 bis 30 Vol.-% Glycerin in Bezug auf das Gesamtvolumen der Zusammensetzung.

2. Konjugatzusammensetzung nach Anspruch 1, wobei die Zusammensetzung in einer Form ausgewählt aus einer wässrigen Lösung, einer Öl-in-Wasser-Emulsion, einer Wasser-in-Öl-Emulsion, einer multiplen Emulsion, einer Mikroemulsion, einer festen Emulsion, einem wässrigen Gel oder einem wässrig-alkoholischen Gel, vorliegt.

3. Verwendung einer Zusammensetzung nach einem der Ansprüche 1 bis 2 in einem Verfahren zum Nachweisen von Verbindungen.

4. Verwendung einer Zusammensetzung nach Anspruch 3 in einem Kit zur Durchführung eines Verfahrens zum Nachweisen von biologischen Verbindungen.

## Claims

1. A composition of conjugates comprising an antibody coupled to an enzyme, fetal calf serum, from 5% to 50% by volume of normal rabbit serum relative to the total volume of the composition, and from 1% to 30% by volume of glycerol relative to the total volume of the composition.

2. The composition of conjugates according to claim 1, said composition being in a form chosen from an aqueous solution, an oil-in-water or water-in-oil emulsion, a multiple emulsion, a microemulsion, a solid emulsion, or an aqueous or aqueous-alcoholic gel.

3. The use of a composition as claimed in any one of claims 1 to 2, in a method for detecting compounds.

4. The use of a composition as claimed in claim 3, in a kit for implementing a method for detecting biological compounds.
